# EUROPEAN PATENT APPLICATION

(11) **EP 4 825 399 A1**
(43) Date of publication of application: **21.10.2026**
(21) Application number: 26173062.6
(22) Date of filing: 17.04.2026
(51) Int. Cl.: A61L 2/08, B65B 55/08, G21K 5/02, H01J 7/00

(54) **ELECTRON BEAM EMITTING DEVICE FOR STERILIZING A WEB OF PACKAGING MATERIAL**

(30) Priority: 17.04.2025 IT 202500008733
(71) Applicant: Tetra Laval Holdings & Finance S.A., 1009 Pully (CH)
(72) Inventor: OMRANE, Alaa, 22186 LUND (SE); CAGLAR, Onur, 3175 Wünnewil-Flamatt (CH); BELLUCCI, Alessandra, 41123 MODENA (IT); CASADEI, Fabrizio, 41123 MODENA (IT)
(74) Representative: Tetra Pak Patent Attorneys

(57) **Abstract**

There is described an electron beam emitting device (11) comprising:
- a main housing (12) internally defining an inner compartment (13) and having an exit window (16);
- a generation member (14) arranged within the inner compartment (13) and configured to emit electrons and to direct the electrons through the exit window (16) and out of the inner compartment (13);
- a protective element (18) arranged at the exit window (16);
the protective element (18) comprises at least two of titanium, tantalum and niobium.

## Description

### TECHNICAL FIELD

The present invention relates to an electron beam emitting device configured to sterilize a web of packaging material for the production of packages containing a pourable product, preferably a pourable food product.

### BACKGROUND ART

As it is generally known, many pourable food products, such as fruit juice, UHT (ultra-high temperature-treated) milk, wine, tomato sauce, etc., are sold in packages made of sterilized packaging material.

A typical example is the parallelepiped-shaped package for pourable food products known as Tetra Brik Aseptic (registered trademark), which is made by folding and sealing a laminated web of packaging material.

The packaging material has a multilayer structure comprising a base layer, e.g. made of paper, covered on both sides with layers of heat-sealable material, e.g. polyethylene.

In the case of aseptic packages for long-storage products, such as UHT milk, the packaging material also comprises a layer of oxygen-barrier material, e.g. an aluminum foil, which is superimposed on a layer of heat-sealable material, and is in turn covered with another layer of heat-sealable material forming the inner face of the package eventually contacting the food product.

Packages of this sort are normally produced on fully automatic packaging machines, which form and fill the packages starting from a multilayer web of packaging material.

In particular, according to a non-limiting example of a packaging machine, a continuous tube is formed from the web of packaging material which is initially wound in a reel and fed through a plurality of unwinding rollers.

The web of packaging material is typically maintained in a closed, sterile environment (defined by an isolation chamber) and, while advanced by the aforementioned unwinding rollers, is folded to form the tube by means of a known web folding unit and then sealed longitudinally.

In order to perform the package forming operations, the tube is continuously fed along a straight vertical direction, is filled with the sterilized food product from above and is formed, sealed and subsequently cut along equally spaced transversal cross-sections extending along a direction orthogonal to the vertical direction, according to a manner known and not described in detail.

So-called pillow packs are obtained thereby, which have a longitudinal sealing band, a top transversal sealing band and a bottom transversal sealing band. The pillow packs are then cut at the cross-sections to be separated from one another and directed to a folding unit of the packaging machine for the final folding thereof.

The finished packages are thereby obtained.

The need for sterilizing the web of packaging material before the forming and filling operations is known in the field, with the aim to extend the shelf-life of the products being packed.

In particular, since the web of packaging material gets into contact with the pourable product, in use, it must be guaranteed that the cleanliness and/or sterility of the isolation chamber and of the web of packaging material meets predetermined standards.

According to a known solution, the advancing web is irradiated with an electron beam emitted by an electron beam emitting device located at a sterilization station.

Typically, the packaging machines comprise two emitting devices, in order to sterilize the two opposite surfaces of the web of packaging material.

Each emitting device typically comprises a main housing internally defining an inner compartment and having an exit window facing the respective surface of the web to be sterilized.

The emitting device essentially comprises a beam generation member or irradiation source, usually defined by a tungsten filament, which is arranged within the inner compartment and is electrically connected to a power supply.

The generation member is configured to generate the electron beam.

In detail, being supplied with electrical energy, the generation filament undergoes a so-called thermionic emission whereby it starts to emit electrons.

The generation member is provided with means for directing the generated electron beam through the exit window and out of the inner compartment, and thus towards the web of packaging material.

The exit window is provided with a protective foil which covers the exit window itself, thereby sealing the inner compartment from the external environment.

The protective foil typically comprises titanium.

The protective foil has an inner surface facing the inner compartment and an outer surface opposite to the inner surface, and therefore facing the web, in use. The outer surface may face away from the inner compartment. The outer surface may face an external environment.

As it is known, the protective foil is configured to protect and divide the inner compartment from the external environment. A need is felt to strengthen the protective foil. In particular, the environment in which the web is advanced at the sterilization station may be quite harsh and the protective foil, especially its external surface, should withstand such environment.

Hence, it must be ensured that the protective foil resists to the (chemical or otherwise) stresses from the external environment.

To this end, the protective foil usually comprises a coating on the outer surface.

In detail, the coating is a corrosion resistant coating, configured to withstand the possibly corrosive environment outside of the inner compartment.

According to a known configuration, the coating comprises rhodium, which significantly improves the anti-corrosion properties of the titanium substrate of the protective foil.

Although the emitting devices of the known type work satisfyingly well, the Applicant has noted that they are still open to further improvement, in particular as per the reduction of their cost while at least maintaining the same anti-corrosion properties.

### DISCLOSURE OF INVENTION

It is therefore an object of the present invention to provide an electron beam emitting device which is designed to meet one or more of the above-mentioned needs in a straightforward and low-cost manner.

This object is achieved by an electron beam emitting device as claimed in the appended independent claim 1.

Preferred embodiments of the present invention are laid down in the appended dependent claims.

### BRIEF DESCRIPTION OF THE DRAWINGS

A non-limiting embodiment of the present invention will be described by way of example with reference to the accompanying drawings, in which:
Figure 1 is a schematic perspective view, with parts removed for clarity, of a packaging machine comprising an electron beam emitting device according to the present invention;
Figure 2 is a larger-scale perspective view of a detail of the packaging machine of Figure 1, with parts removed for clarity;
Figure 3 is a schematic perspective view of an emitting device of the packaging machine; and
Figure 4 is a schematic sectioned view of an exit window of the emitting device of Figure 3.
Figure 5 shows FIB/FEG-SEM images of corrosion drop tests with 30% HNO₃ on (1) uncoated Ti foil (Figure 5A), (2) Rh-coated Ti foil (thickness 80 nm) (Figure 5D), (3) TiTaNb-coated Ti foil (thickness 30 nm) (Figure 5F), (4) TiTaNb-coated Ti foil (thickness 100 nm) (Figure 5E), (5) TiTaNb-coated Ti foil (thickness 290 nm) (Figure 5B), and (6) TiTaNb-coated Ti foil (thickness 300 nm) (Figure 5C). Arrows indicate the coating thickness, while circles indicate pits caused by corrosion.

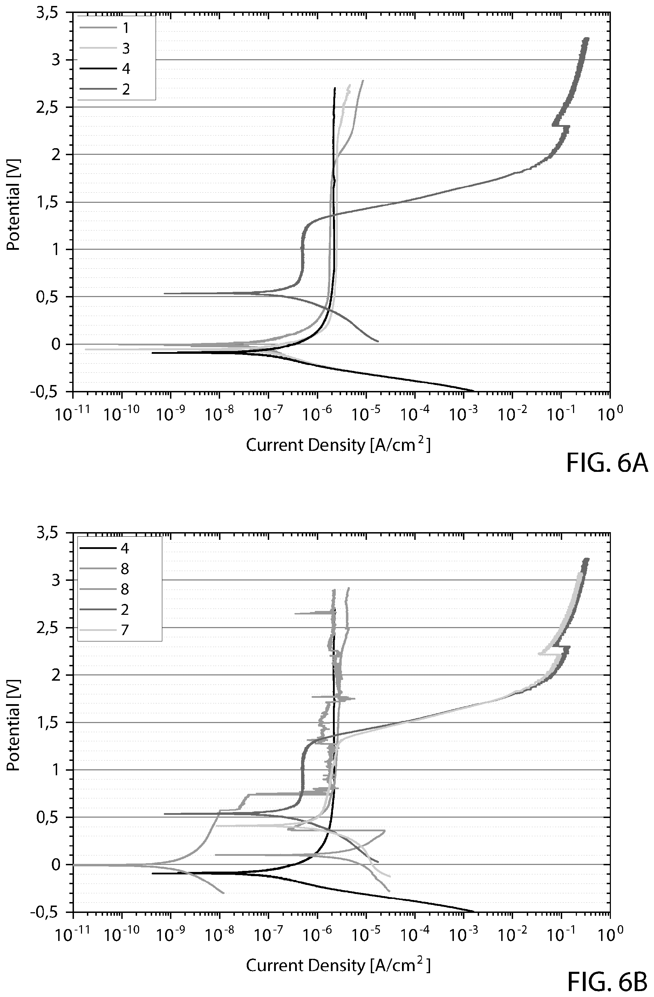

Figure 6 shows polarization curves for potentiodynamic polarization tests in 6% HNO₃, with the x axis representing current density (A/cm²) and the y axis representing potential (V). Figure 6A reports polarization curves for (1) uncoated Ti foil, (2) Rh-coated Ti foil (80 nm thickness), (3) TiTaNb-coated Ti foil (30 nm thickness), and (4) TiTaNb-coated Ti foil (100 nm thickness). Figure 6B reports polarization curves for (2) Rh-coated Ti foil (80 nm thickness), (4) TiTaNb-coated Ti foil (100 nm thickness), (7) Rh-coated Ti foil (80 nm thickness) with an induced crease, and (8) TiTaNb-coated Ti foil (100 nm thickness) with an induced crease (2 duplicates).

### BEST MODE FOR CARRYING OUT THE INVENTION

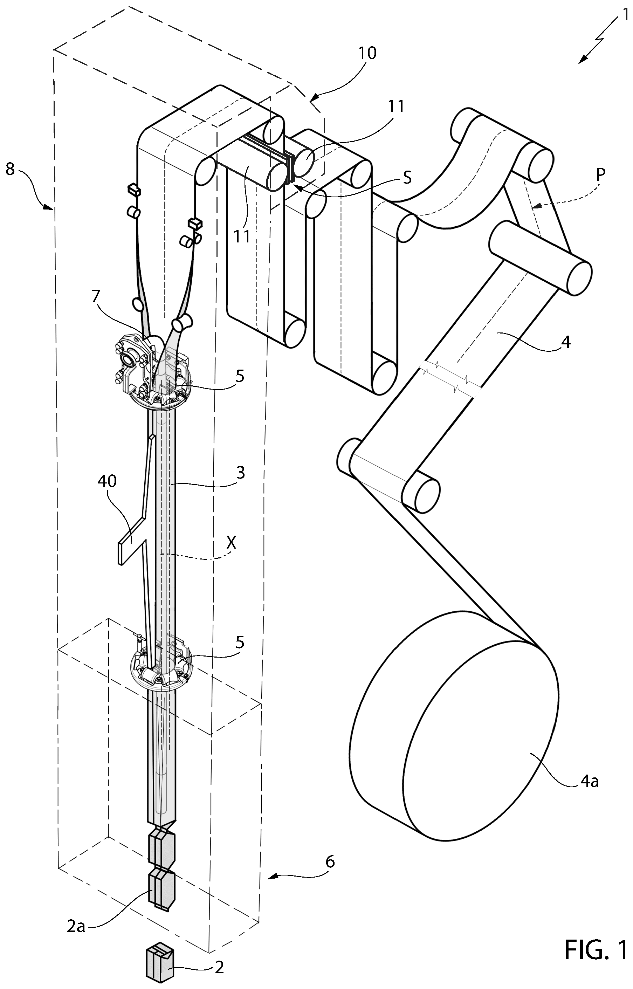

With reference to Figure 1, number 1 indicates as a whole a non-limiting example of a packaging machine configured to produce sealed packages 2 containing a pourable product, preferably a pourable food product such as pasteurized or UHT milk, water, fruit juice, wine, peas, beans, etc.

The present invention will refer to an electron beam emitting device for the use in such a packaging machine 1, without however losing on generality. In fact, the electron beam emitting device as described hereinafter may be used and/or implemented in other apparatuses or machines different from packaging machine 1, which is hereby described as a mere example of application for the emitting device.

Packaging machine 1 is configured to form, seal and fold packages 2 starting from a web 4 of packaging material, which is initially wound in a reel 4a, and then folded into a tube 3 of packaging material.

As shown in Figure 1, packaging machine 1 comprises:
- conveying means configured to advance web 4 along an advancement path P;
- a tube folding device 5 configured to progressively fold web 4 into a tube 3;
- a sealing element 40 for longitudinally sealing tube 3, in a manner known and not described in detail;
- a filling device 7 for filling tube 3 (from above) with the pourable product;
- a forming and sealing unit 6 (only schematically shown) configured to form tube 3 and seal tube 3 along successive transversal cross-sections thereof in order to obtain a sequence of pillow packs 2a, in a manner known and not described in detail;
- an isolation chamber 8 internally defining an environment containing a controlled atmosphere, in particular sterile and/or aseptic gas, preferably sterile and/or aseptic air, and housing at least the forming and sealing unit 6, the tube folding device 5, the sealing element 40 and at least part of the filling device 7;
- a folding device (not shown) configured to sequentially fold packs 2a in order to obtain a plurality of sealed packages 2.

Tube folding device 5 and sealing element 40 define together a tube forming unit.

Preferably, forming and sealing unit 6 has an axis X along which tube 3 is fed, in use.

Axis X is parallel to a straight direction, which preferably is a straight vertical direction.

Hence, in use, tube 3 is fed along axis X, downwards, and while being filled from above is formed and sealed by forming and sealing unit 6.

More specifically, tube 3 is drawn (downwards) along axis X by forming and sealing unit 6 in a known manner.

In this way, a plurality of pillow packs 2a are obtained.

In light of the above, packaging machine 1 is configured to form and seal a plurality of pillow packs 2a containing the pourable product starting from web 4 and tube 3 and then to fold the pillow packs 2a for obtaining the aforementioned formed, sealed and folded packages 2 containing the pourable product.

As schematized in Figure 1, packaging machine 1 further comprises a sterilization unit 10 arranged at a sterilization station S along advancement path P, in a position upstream of tube folding device 5 and configured to perform a sterilizing treatment on web 4.

In particular, sterilization unit 10 is configured to sterilize web 4 by means of electron beam irradiation.

To this end, sterilization unit 10 comprises at least one electron beam emitting device 11.

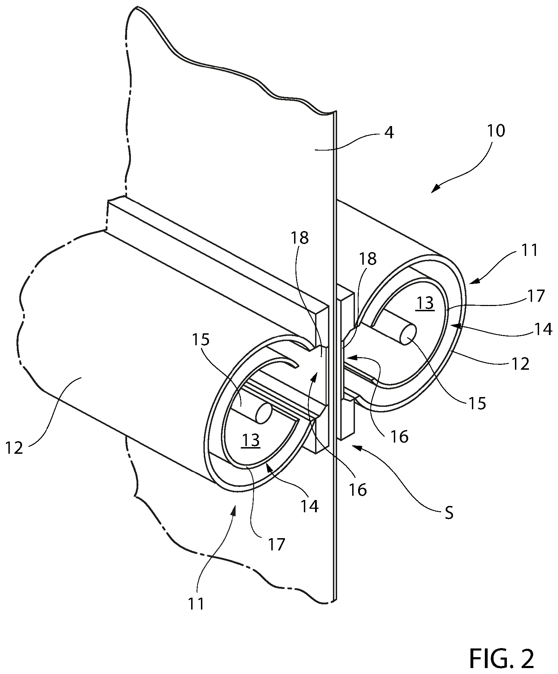

Preferably, sterilization unit 10 comprises a pair of emitting devices 11 (as shown in Figures 1 and 2), arranged at opposite sides of web 4 at sterilization station S, for irradiating web 4 from both sides in order to obtain the desired level of sterilization.

In light of the above, each emitting device 11 is configured to sterilize the packaging material for the production of packages 2.

The web 4 crossing, in use, sterilization station S defines, thus, a target to be sterilized by the emitting devices 11.

Reference will be made in the following to a single emitting device 11. However, the structural and functional features described therefor are equally applicable to the other emitting device 11.

Emitting device 11 comprises a main housing 12 which internally defines an inner compartment 13.

Inner compartment 13 defines, in turn, a generation chamber wherein the electron beam is generated, as explained below.

Emitting device 11 further comprises a generation member 14 arranged within inner compartment 13 and configured to emit electrons.

In detail, generation member 14 includes a filament 15 of electrically conductive material, preferably tungsten.

Filament 15 extends within inner compartment 13 and defines the irradiation source of emitting device 11.

More specifically, filament 15 is supplied, in use, with electrical energy (from a power source) and heats up due to Joule effect. When filament 15 reaches a predetermined temperature, a so-called thermionic emission is established. Thermionic emission is the liberation of charged particles from a hot electrode whose thermal energy gives some particles enough kinetic energy to escape the electrode surface.

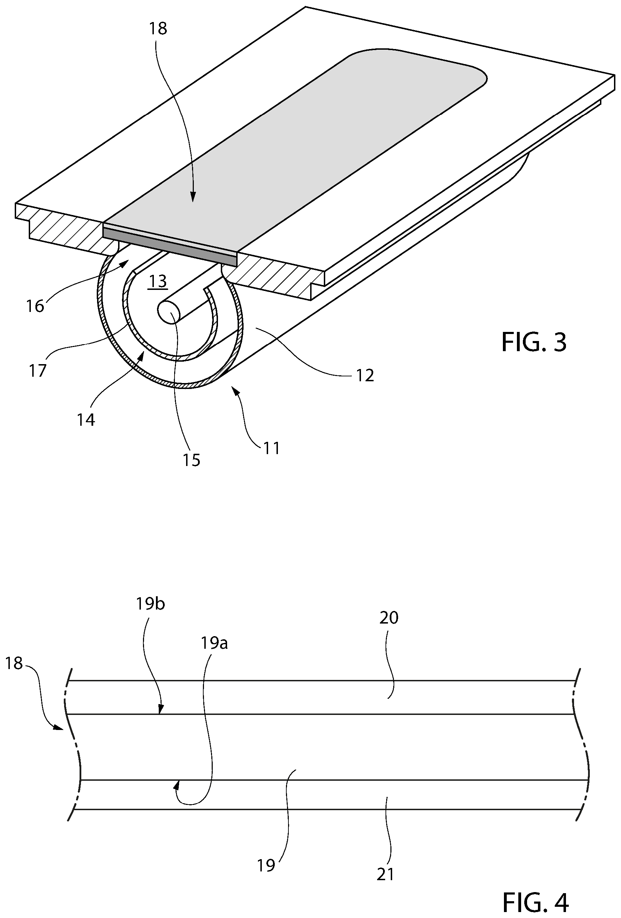

As better visible in Figure 3, main housing 12 includes an exit window 16.

Exit window 16 is basically defined by an elongated slit obtained in the main housing 12 and is arranged facing web 4 at sterilization station S, as shown in Figure 2.

Generation member 14 further comprises a deflector member 17 arranged within the inner compartment 13 and partially surrounding filament 15, with the exception of an angular region around filament 15 angularly corresponding (i.e. angularly aligned) to the exit window 16.

Deflector member 17 is configured to direct the electrons generated (emitted) by the filament 15 through the exit window 16 and out of the inner compartment 13. The emitted electrons are directed to the target, e.g. towards web 4 transiting at sterilization station S.

In light of the above, generation member 14 as a whole is configured to emit electrons and to direct the electrons through the exit window 16 and out of the inner compartment 13.

As best visible in Figure 3, emitting device 11 further comprises a protective element 18 arranged at exit window 16.

In particular, the protective element comprises, and preferably consists of, a protective foil 18 covering the exit window 16, to which the present invention will refer to, without however losing on generality.

More in particular, the protective foil 18 allows the passage of electrons, while preventing the entrance of undesired substances (including external air) within inner compartment 13.

More generally, protective element 18 is defined by a multilayer structure, preferably in the shape of a foil.

It must be ensured that the protective element 18 resists to (chemical) stresses from the environment at the sterilization station S.

Advantageously, protective element 18 comprises at least two between titanium, tantalum and niobium, preferably titanium and at least one of tantalum and niobium, most preferably all of titanium, tantalum and niobium.

More specifically, according to an important aspect of the present invention, the aforementioned multilayer structure comprises, as schematized in Figure 4:
- a substrate 19 comprising titanium, preferably made of titanium;
- a coating 20 comprising at least two of titanium, tantalum and niobium, preferably titanium and at least one of tantalum or niobium, most preferably titanium (Ti), tantalum (Ta) and niobium (Nb),
- preferably a thermally conductive layer 21.

In particular, coating 20 is an alloy of Titanium, comprising Tantalum and/or Niobium. Herein, alloy has the conventional meaning that is found in the art, and is defined as a mixture of two or more elements, where at least one is a metal. An alloy of Titanium is intended to be an alloy wherein Titanium is the major component. Preferably, Titanium is at least 75 wt.% of the alloy, more preferably at least 80 wt.% of the alloy.

Coating 20 is intended for coating substrate 19 comprising Titanium, preferably entirely made of Titanium. In some preferred embodiments, substrate 19 is a Titanium foil. The inventors have found that a coating 20 being an alloy of Titanium, preferably comprising at least 75 wt.%, more preferably 80 wt.% Titanium, has improved affinity with substrate 19 than a coating comprising lower amounts of Titanium. It is preferred that the amount of Tantalum and/or Niobium in the alloy is kept below a total of Tantalum and/or Niobium of 20 wt.% of the alloy. The inventors have observed that a higher amount of Tantalum and/or Niobium leads to cracking or delamination between coating 20 and substrate 19. Without wishing to be bound by a theory, this is believed to be due to the formation of intermetallic states.

Furthermore, the inventors have surprisingly found that a high amount of Titanium in the alloy ensures sufficient transparency to the electron beam. To ensure transparency to the electron beam it is preferred that the amount of Tantalum and/or Niobium in the alloy is kept low, e.g., below a total of Tantalum and/or Niobium of 20 wt.% of the alloy. Preferably, Tantalum is less than 15 wt.%, more preferably less than 10 wt.%, even more preferably less than 5 wt.% of the alloy. Preferably, Niobium is less than 15 wt.%, more preferably less than 10 wt.%, even more preferably less than 5 wt.% of the alloy. Without wishing to be bound by a theory, the lower density of Titanium (around 4.5 g/cm³) compared to Tantalum (around 16.7 g/cm³) and Niobium (8.6 g/cm³) ensures better transparency to the electron beams. The inventors have surprisingly found that an amount of at least 75 wt.%, more preferably at least 80 wt.% of Titanium and at most 20 wt.% of Tantalum and/or Niobium ensured optimal transparency to the electron beam.

Preferably, coating 20 is entirely made of at least two of titanium, tantalum and niobium. More preferably, coating 20 is entirely made of titanium and at least one of tantalum or niobium. Most preferably, coating 20 is entirely made of titanium, tantalum and niobium.

When coating 20 comprises or is entirely made of titanium and tantalum, coating 20 preferably has a composition comprising, in weight percentage:
- Ti between 80% and 99%, more preferably between 85% and 95%;
- Ta between 1% and 15%, more preferably between 3% and 13%;
the balance being impurities.

When coating 20 comprises or is entirely made of titanium and niobium, coating 20 preferably has a composition comprising, in weight percentage:
- Ti between 80% and 99%, more preferably between 85% and 95%;
- Nb between 1 and 15%, more preferably between 1% and 5%;
the balance being impurities.

In a preferred embodiment, coating 20 comprises or is entirely made of titanium, tantalum and niobium, and has a composition comprising, in weight percentage:
- Ti between 80% and 99%, more preferably between 85% and 95%;
- Ta between 1% and 15%, more preferably between 3% and 13%;
- Nb between 1 and 15%, more preferably between 1% and 5%;
the balance being impurities.

Most preferably, coating 20 has a composition comprising, in weight percentage:
- Ti substantially 90%;
- Ta substantially 8%;
- Nb substantially 2%;
the balance being impurities.

In a most preferred embodiment, coating (20) is an alloy of Titanium, comprising Tantalum and Niobium, wherein Titanium is 75 - 95%, more preferably 80 - 95%, by weight of the alloy; and wherein the sum of Tantalum and Niobium is 1 - 20%, more preferably 1 - 15% by weight of the alloy. This configuration has the advantages of achieving corrosion protection, while avoiding cracking and delamination due to the formation of intermetallic state, and being transparent to the electron beam.

Substrate 19 has an inner surface 19a facing the inner compartment 13 and an outer surface 19b opposite to the inner surface 19a.

Advantageously, coating 20 is applied onto the outer surface 19b.

Preferably, a thermally conductive layer 21 is applied onto the inner surface 19a. The primary function of layer 21 is to convey and dissipate heat from the protective element 18. Preferably, thermally conductive layer 21 is comprises aluminium (Al), more preferably thermally conductive layer 21 is made of aluminium (Al).

The advantages of emitting device 11 according to the present invention will be clear from the foregoing description.

In particular, thanks to the presence, and most importantly to the composition of the coating 20, which includes titanium, tantalum and/or niobium, the resistance of protective element 18 to (chemical) stresses is improved. In detail, the anti-corrosion properties of the protective element 18 are ensured and improved compared to the known rhodium coatings. Moreover, the usage of rhodium, which is relatively expensive, can be avoided.

Preferably, coating 20 is free of noble metals or, in other words, does not comprise any noble metal. As well-known in the art, a noble metal is a metallic element characterized by very low chemical reactivity, in particular a high resistance to oxidation and corrosion under normal and elevated environmental conditions, and is usually found in nature in its raw form (with oxidation state 0). Herein, noble metals are intended in their strict chemical definition, namely noble metals are gold, platinum, ruthenium, rhodium, palladium, osmium, and iridium. Herein free of noble metals is intended as comprising less than 1%, preferably 0 - 0.5%, more preferably 0 - 0.05 wt.%, of (the total sum of) noble metals, by weight of the coating. Hence, an effective and economical protective foil 18 can be obtained thanks to the coating 20 comprising at least two of titanium, tantalum and niobium (instead of rhodium), and in particular having the aforementioned composition.

Preferably, coating 20 is applied onto substrate 19 by means of Physical Vapor Deposition, or PVD.

In this way, the coating 20 applied by Physical Vapor Deposition (PVD) enhances surface properties such as wear resistance and corrosion protection. In fact, the PVD process involves the deposition of thin films, which bond to the substrate at the atomic level, resulting in a durable, high-performance coating that can withstand harsh environments and extend the lifespan of the material.

Preferably, coating 20 applied by PVD is homogeneous.

Preferably, coating 20 has a thickness of 20 - 350 nm. In a preferred embodiment, coating 20 has a thickness of 30 - 150 nm, more preferably 80 - 120 nm. In another preferred embodiment, coating 20 has a thickness of 200 - 330 nm. Clearly, changes may be made to emitting device 11 as described herein without, however, departing from the scope of protection as defined in the accompanying claims.

### EXAMPLES

The resistance to corrosion of different coatings was tested in the following examples.

### Example 1 - Initial screening

Coatings of 1 µm thickness were deposited on a steel plate. The coatings contained Rh (control), TiTa, TiNb, or TiTaNb. Corrosion was evaluated by exposure to NaCl. The coatings containing TiTa, TiNb, or TiTaNb showed improved resistance to corrosion (data not shown).

### Example 2 - Drop tests

Corrosion resistance was qualitatively evaluated by drop test. The following samples were evaluated:
(1) uncoated Ti foil;
(2) Ti foil coated with a Rh layer (80 nm thickness);
(3) Ti foil coated with a TiTaNb layer (30 nm thickness);
(4) Ti foil coated with a TiTaNb layer (100 nm thickness);
(5) Ti foil coated with a TiTaNb layer (290 nm thickness);
(6) Ti foil coated with a TiTaNb layer (300 nm thickness).

The TiTaNb coatings were achieved by physical vapor deposition of a target of 80 wt.% TiTa and 20 wt.% TiNb for 600 seconds (sample 3), 900 seconds (sample 4), 1500 seconds (sample 5), or 1800 seconds (sample 6).

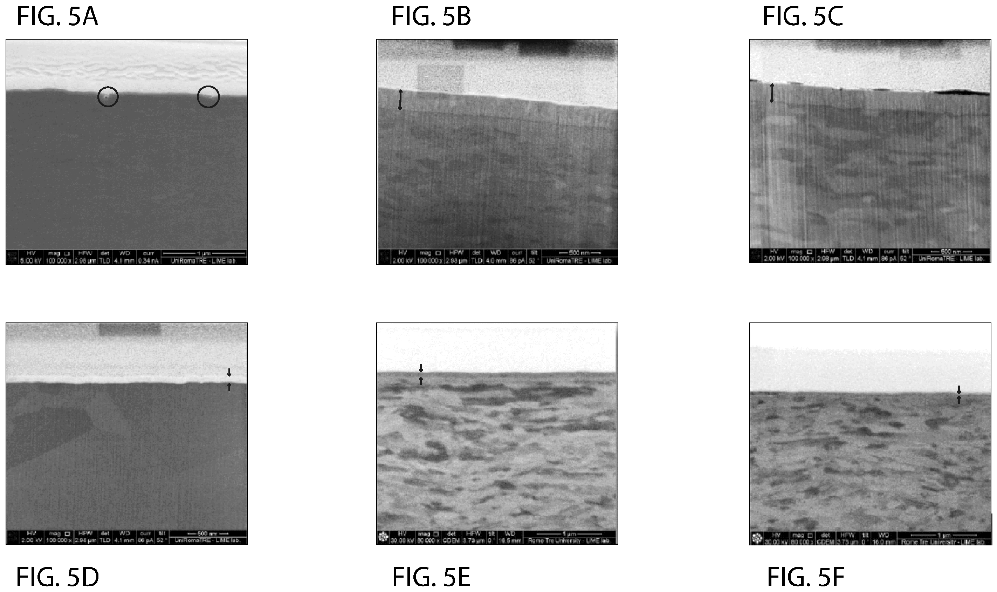

All samples were challenged by drops of 30% HNO₃ at 70 °C. Images of the samples after HNO₃ exposure were taken by FIB/FEG-SEM. As shown in Figure 5, HNO₃ drops resulted in pits of around 80 nm, indicated by circles in Figure 5A, on the uncoated Ti foil (sample 1). In contrasted, the Rh coating (sample 2, Figure 5D) and the TiTaNb coatings (samples 3-6, Figures 5B-C and E-F) prevented pit formation. Furthermore, no delamination between the coatings and the Ti foil was observed (Figures 5B-F).

The drop tests results indicate that the TiTaNb coating of the invention is at least as good as the known Rh coating in preventing corrosion, with the added advantage of reduced costs.

### Example 3 - Potentiodynamic polarization tests

Corrosion resistance was qualitatively evaluated by potentiodynamic polarization tests. The setup included a three electrodes cell configuration with an Autolab PGSTAT302N and vs the 6.0733.100 Autolab reference electrode (Ag/AgCl in KCl 3M). After an initial open circuit potential measurement (22 hours), a scan in potential (from 0 to -0.4 V vs OCP and from -0.4 to 2.8 V vs OCP) was performed at a scan rate of 0.4 mV/s. Being HNO₃ a highly aggressive/oxidizing agent, concentrations and temperatures were defined aiming at having a clearly readable curve and, at the same time, an easy handling. 6% HNO₃ showed relevant effects in terms of OC potential variation overtime.

The samples tested were
(1) uncoated Ti foil;
(2) Ti foil coated with a Rh layer (80 nm thickness);
(3) Ti foil coated with a TiTaNb layer (30 nm thickness);
(4) Ti foil coated with a TiTaNb layer (100 nm thickness).

The TiTaNb coatings were achieved by physical vapour deposition of a target of 80 wt.% TiTa and 20 wt.% TiNb, for 600 seconds (sample 3) or 900 seconds (sample 4).

Two further sample were prepared and included an induced crease, which mimics mechanical deformations and defects that may occur in the protective element, while the filling machine is running. The two additional sample were
(7) Ti foil coated with a Rh layer (80 nm thickness) with an induced crease, i.e. creased sample 2; and
(8) Ti foil coated with a TiTaNb layer (100 nm thickness) with an induced crease (2 duplicates), i.e. creased sample 4.

Figure 6 reports polarization curves for the tested samples at 6% HNO₃. Initially, the corrosion potential and corrosion current of the TiTaNb-coated Ti foils (samples 3 and 4) are similar to those of uncoated Ti foil (sample 1), while the corrosion potential of Rh-coated Ti foil (sample 2) is increased, due to noble metal behaviour of Rh (Figure 6A). However, samples 3 and 4 (TiTaNb coatings) result in an increased passive region, where corrosion current does not increase with increasing potential (Figure 6A). This lack of increase in corrosion current indicates a lack of corrosion by HNO₃. In contrast, sample 1 (uncoated Ti) and sample 2 (Rh coating) show a smaller passive region than samples 3 and 4 (TiTaNb coatings). The same effect on the passive region is observed in samples 7 and 8 having an induced crease (Figure 6B). The signal-to-noise ratio is worse for samples 7 and 8 due to the mechanical deformation. Nevertheless, it is possible to observe in Figure 5B a larger passive region for both duplicates of sample 8 than for sample 7. Taken together, these results indicate that passivation to corrosion is improved for the TiTaNb-coated Ti foils compared to the Rh-coated Ti foils. Such improved corrosion passivation is maintained even in the presence of mechanical deformations or defects, that may occur during filling machine performance.

### Example 4 - Quantification of metal concentrations

In this example, the concentrations of each metals in a TiTaNb coating is quantified. A sample of Ti foil coated with TiTaNb was prepared. The TiTaNb coating was achieved by physical vapour deposition of a target of 80 wt.% TiTa and 20 wt.% TiNb, for 900 seconds. The sample resembled sample 4 of Example 3. The sample was analysed by Energy-dispersive X-ray spectroscopy (EDS) elemental analysis. Two different approaches were employed for the determination of Ta and Nb concentrations: quantification of Ta and Nb considering all the detected elements, and quantification of Ta and Nb considering only the coating elements. The results are reported in Table 1.

**Table 1 - results from EDS on the sample of Example 4**

| | Wt.% based on all elements | Wt.% based on coating elements |
|---|---|---|
| Ti | 82.13 ± 0.16 | 88.63 ± 0.33 |
| Ta | 8.34 ± 0.29 | 8.95 ± 0.29 |
| Nb | 2.25 ± 0.13 | 2.42 ± 0.14 |
| C | 1.33 ± 0.04 | - |
| O | 5.96 ± 0.19 | - |

The coating was inspected by SEM and appeared homogenous without significant cracks, pits, or delamination. The coating thickness was determined to be 100 µm.

## Claims

1. Electron beam emitting device (11) comprising:
- a main housing (12) internally defining an inner compartment (13) and having an exit window (16);
- a generation member (14) arranged within the inner compartment (13) and configured to emit electrons and to direct the electrons through the exit window (16) and out of the inner compartment (13);
- a protective element (18) arranged at the exit window (16);
wherein the protective element (18) comprises, and in particular consists of, a foil covering the exit window (16) ;
wherein the protective element (18) is defined by a multilayer structure comprising:
- a substrate (19) comprising titanium; and
- a coating (20), wherein the coating (20) is an alloy of titanium, comprising tantalum and/or niobium.

2. Electron beam emitting device (11) as claimed in claim 1, wherein coating (20) comprises more than 75 wt.%, preferably more than 80 wt.% titanium.

3. Electron beam emitting device (11) as claimed in claim 1 or 2, wherein coating (20) comprises less than 20 wt.% of tantalum and/or niobium.

4. Electron beam emitting device (11) as claimed in any one of the preceding claims, wherein the coating (20) comprises titanium, tantalum, and niobium.

5. Electron beam emitting device (11) as claimed in any one of the preceding claims, wherein substrate (19) is a titanium foil.

6. Electron beam emitting device (11) as claimed in any one of the preceding claims, wherein coating (20) is free of noble metals.

7. Electron beam emitting device (11) as claimed in any one of the preceding claims, wherein coating (20) comprises less than 1 wt.% noble metals.

8. Electron beam emitting device (11) as claimed in any one of the preceding claims, wherein coating (20) has a thickness of 30 - 150 nm, preferably 80 - 120 nm.

9. Electron beam emitting device (11) as claimed in any one of claims 1 - 7, wherein coating (20) has a thickness of 200 - 330 nm.

10. Electron beam emitting device (11) as claimed in any one of the preceding claims, wherein the coating (20) has a composition comprising, in weight percentage:
- Ti between 80% and 99%;
- Ta between 1% and 15%;
- Nb between 1% and 15%;
the balance being impurities.

11. Electron beam emitting device (11) as claimed in any one of the preceding claims, wherein the coating (20) has a composition comprising, in weight percentage:
- Ti between 85% and 95%;
- Ta between 3% and 13%;
- Nb between 1% and 5%;
the balance being impurities.

12. Electron beam emitting device (11) as claimed in any one of the preceding claims, wherein the coating (20) is applied onto the substrate (19) by means of Physical Vapor Deposition or PVD.

13. Electron beam emitting device (11) as claimed in any one of the preceding claims, wherein the substrate (19) has an inner surface (19a) facing the inner compartment (13) and an outer surface (19b) opposite to the inner surface (19a);
wherein the coating (20) is applied onto the outer surface (19b).

14. Sterilization unit (10) for sterilization, comprising at least one electron beam emitting device (11) as claimed in any one of the preceding claims.

15. Packaging machine (1) configured for producing packages (2) containing a pourable product starting from a web (4) of packaging material, the packaging machine (1) comprising:
- conveying means configured to advance the web (4) along an advancement path (P);
- a tube forming unit (5, 40) configured to fold the web (4) into a tube (3) and to longitudinally seal the tube (3);
- a forming and sealing unit (6) configured to form the tube (3) and seal the tube (3) along successive transversal cross-sections thereof in order to obtain a sequence of packs (2a);
- a folding device configured to sequentially fold the packs (2a) in order to obtain a plurality of sealed packages (2); and
- a sterilization unit (10) according to claim 14 arranged along the advancement path (P) upstream of the tube forming unit (5, 40) and configured to sterilize the web.
